(19) Europäisches Patentamt
European Patent Office
Office européen des brevets

(11) Numéro de publication: **0 203 865 B1**

# (12) FASCICULE DE BREVET EUROPEEN

(45) Date de publication de fascicule du brevet: **25.03.92**

(51) Int. Cl.[5]: **A61K 35/16**, A61M 1/36

(21) Numéro de dépôt: **86401115.0**

(22) Date de dépôt: **27.05.86**

Le dossier contient des informations techniques présentées postérieurement au dépôt de la demande et ne figurant pas dans le présent fascicule.

## (54) Procédé de séparation des anticorps antifacteur VIII:C, utilisable notamment pour l'épuration du plasma sanguin d'un hemophile de type A.

(30) Priorité: **31.05.85 FR 8508227**

(43) Date de publication de la demande: **03.12.86 Bulletin 86/49**

(45) Mention de la délivrance du brevet: **25.03.92 Bulletin 92/13**

(84) Etats contractants désignés: **BE CH DE GB IT LI NL SE**

(56) Documents cités:
**FR-A- 2 548 193**

**CHEMICAL ABSTRACTS, vol. 91, no. 7, 13 août 1979, Columbus, OH (US); H.LEE et al., p. 488, no. 54297z**

**CHEMICAL ABSTRACTS, vol. 101, no. 7, 13 août 1984, Columbus, OH (US); I.M.NILSSON et al., p. 451, no. 52882n**

(73) Titulaire: **CIS BIO INTERNATIONAL**
**RN 306**
**F-91000 Saclay(FR)**

(72) Inventeur: **Belattar, Noureddine**
**Foyer/Hotel Rue G. Lacassagne**
**F-94120 Fontenay Sous Bois(FR)**
Inventeur: **Gulino, Danielle**
**4, Allée des Jonquilles**
**F-93360 Neuilly-Plaisance(FR)**
Inventeur: **Jozefonvicz, Jacqueline**
**65, Deuxième Avenue**
**F-60260 Lamorlaye(FR)**

(74) Mandataire: **Pottier, Pierre Société BREVATOME et al**
**25, Rue de Ponthieu**
**F-75008 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

La présente invention a pour objet un procédé de séparation des anticorps AntiVIII:C présents dans un liquide tel que du plasma sanguin.

De façon plus précise, elle concerne des supports en polymère ou en copolymère insolubles, utilisables pour épurer sélectivement les anticorps spécifiques du facteur VIII:C, soit les anticorps AntiVIII:C, présents dans du plasma sanguin tel que celui d'un patient atteint d'hémophilie de type A. En effet, dans le cas de patients atteints d'hémophilie A, ayant eu des transfusions de sang répétées, il se développe généralement des anticorps AntiVIII:C qui neutralisent l'activité coagulante du facteur VIII:C transfusé. La présence de ces anticorps conduit à des difficultés sérieuses. Aussi, pour traiter les hémorragies chez les hémophiles A, il est généralement nécessaire de procéder à des échanges du plasma sanguin, suivis immédiatement par des injections de facteur VIII:C afin d'obtenir une hémostase normale, et ce traitement doit être poursuivi quelquefois pendant de longues périodes selon l'état du patient. Ceci pose certains problèmes, car il est difficile dans ces conditions de maintenir aux taux voulus, les constituants indispensables du plasma sanguin du malade. Par ailleurs, les risques d'infection par des virus tels que ceux de l'hépatite ou le virus LAV/HTLV3 deviennent de plus en plus importants avec ces transfusions répétées.

Aussi, depuis quelques années, les recherches se sont portées sur des procédés d'épuration directe du plasma par adsorption des éléments gênants sur un support approprié. Dans le cas de malades atteints d'hémophilie B, on a pu réaliser cette épuration par adsorption des anticorps spécifiques du facteur IX, soit des anticorps AntiIX, sur une protéine A liée par covalence à de l'agarose, comme cela est décrit par I.M. Nilsson et al. dans Blood, vol.58, n° 1 (juillet 1981), pages 38 à 44. La protéine A réagit avec la partie Fc des immunoglobulines, et lorsqu'elle est liée au sépharose, elle peut être utilisée comme un immunosorbant pour isoler les immunoglobulines G et en conséquence, l'Antifacteur IX. Cependant, la teneur en immunoglobuline totale du plasma ainsi traité ne représente plus que le 1/5 de sa valeur d'origine, ce qui pose certains problèmes. Aussi, les recherches actuelles se sont développées sur des immunosorbants capables de séparer sélectivement les anticorps voulus, notamment ceux présents dans le sang des patients atteints d'hémophilie.

Dans le cas des anticorps AntiVIII:C les recherches actuelles n'ont pas permis d'obtenir une séparation sélective et quantitative de ces anticorps. En effet, on a envisagé de réaliser cette séparation par adsorption sélective au moyen d'un gel chromatographique sur lequel est immobilisé le facteur VIII:C. Cependant, cette méthode est inapplicable car le facteur VIII:C perd très rapidement ses propriétés antigéniques.

Aussi, depuis quelques années, les recherches se sont portées sur l'utilisation de supports inertes sur lesquels sont fixés des composés appropriés permettant de conférer au support une affinité particuliére, par exemple pour certains constituants du sang. Ainsi, le brevet français 83/10773 déposé le 29 juin 1983 par le C.E.A. illustre l'utilisation de polymères de ce type pour séparer et purifier la thrombine. Dans ce cas, on fixe sur le polymère ou copolymère de l'arginine, de la nitroarginine ou un de leurs dérivés tels que l'ester méthylique, pour conférer au support cette affinité vis-à-vis de la thrombine.

La présente invention a précisément pour objet un procédé de séparation des anticorps AntiVIII:C présents dans un liquide au moyen de supports du même type, qui peut être utilisé pour l'épuration plasmatique du sang de malades atteints d'hémophilie A, car il permet de séparer sélectivement les anticorps AntiVIII:C des autres immunoglobulines G.

Le procédé selon l'invention de séparation des anticorps AntiVIII:C présents dans un liquide, consiste :

a)à mettre en contact ledit liquide avec un support solide constitué par un polymère ou un copolymère comportant dans sa chaîne des groupes substituables dont une partie au moins est substituée par des groupements de formule $-(SO_3)_xM$ et $-SO_2Y$, ou $-(SO_3)_xM$ uniquement, dans lesquelles M représente un métal ne réagissant pas avec le liquide et x représente la valence de M, et Y représente un radical obtenu par enlèvement d'un atome d'hydrogène sur la fonction amine d'un composé choisi parmi l'acide glutamique, l'hydroxyproline, la thréonine, et leurs dérivés, et

b)à séparer ensuite le liquide du support sur lequel ont été adsorbés les anticorps AntiVIII:C.

De préférence, le radical Y est un radical provenant de l'acide glutamique, de l'hydroxy proline ou de la thréonine

Selon l'invention, lorsque l'acide $\alpha$-aminé précurseur du radical Y comprend plusieurs fonctions amines, la fonction amine de cet acide $\alpha$-aminé ou de son dérivé, sur laquelle on enlève un atome d'hydrogène pour former le radical Y est la fonction amine située en $\alpha$.

Selon l'invention, les groupements $-SO_2Y$, $-(SO_3)_xM$ peuvent être fixés sur le polymère par l'intermédiaire de groupements ou de chaînes hydrocarbonées.

Selon l'invention, les polymères et copolymères susceptibles d'être utilisés sont des matériaux solides qui comportent le long de leur chaîne des groupes substituables susceptibles de réagir avec l'acide

chlorosulfonique.

A titre d'exemple de tels polymères, on peut citer le polystyrène réticulé, les esters cellulosiques, les éthers cellulosiques, l'acétate de polyvinyle, le chlorure de polyvinyle, le polyisoprène et le polybutadiène. On peut également utiliser des copolymères de styrène, des copolymères d'acétate de vinyle, des copolymères de chlorure de vinyle, des copolymères d'isoprène et des copolymères de butadiène. On précise, par ailleurs, que l'on entend également par copolymère, les copolymères greffés obtenus par greffage d'un monomère vinylique sur un polymère de base, par exemple les copolymères de styrène constitués par un polymère résistant à la chlorosulfonation greffé par du styrène. Dans ce cas, le polymère de base résistant à la chlorosulfonation peut être choisi parmi les polyoléfines, les polymères fluorés et le chlorure de polyvinyle.

De préference, on utilise le styrène ou un copolymère de styrène et, dans ce cas, les groupements fixés sur le polymère ou copolymère sont généralement de deux types, d'une part des groupements de formule $SO_2Y$ dans laquelle Y a la signification donnée ci-dessus et, d'autre part, des groupements de formule $-(SO_3)_xM$ dans laquelle M représente un métal ne réagissant pas avec le liquide et x représente la valence du métal M. Généralement, M est le sodium.

Les supports de l'invention dans lesquels le polymère de base est du styrène ou un copolymère de styrène peuvent être obtenus par un procédé comprenant une première étape de chlorosulfonation du polymère ou du copolymère par réaction de celui-ci avec de l'acide chlorosulfonique, et une deuxième étape consistant à transformer au moins une partie des groupements $-SO_2Cl$ fixés sur le polymère ou le copolymère en groupements $-SO_2Y$.

Lorsque l'on utilise le polystyrène, la réaction de chlorosulfonation correspond au schéma réactionnel suivant :

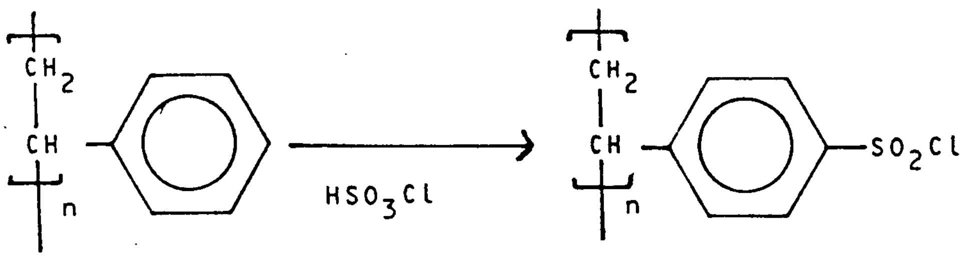


Cette réaction peut être effectuée dans un milieu organique constitué avantageusement par un solvant chloré tel que le dichlorométhane auquel on ajoute du nitrométhane.

Dans la deuxième étape, on transforme les groupes chlorosulfonyle $-SO_2Cl$ en groupes Y par réaction avec l'acide $\alpha$-aminé correspondant qui répond à la formule :

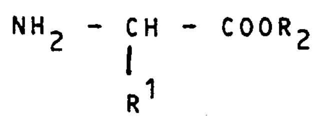

dans laquelle $R^1$ représente la chaîne latérale de l'acide $\alpha$-aminé et $R^2$ représente un atome d'hydrogène ou un radical alkyle. Cette réaction correspond au schéma réactionnel suivant :

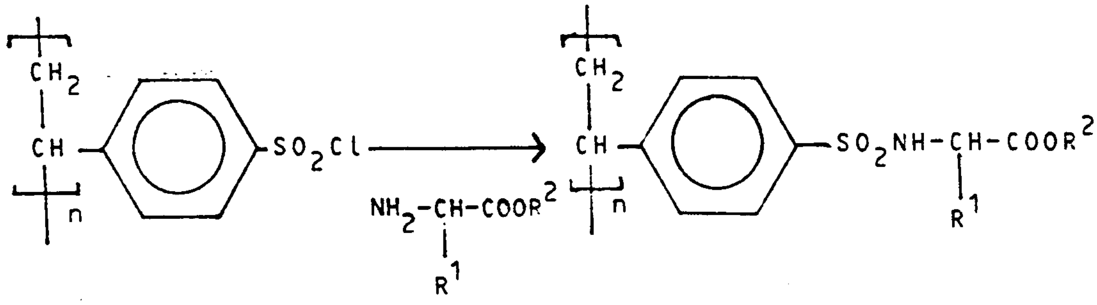

Elle est effectuée généralement dans un milieu contenant le mélange eau-dioxane à la température ambiante lorsque $R^2$ représente un atome d'hydrogène, et dans un milieu contenant du dichlorométhane à une température légèrement supérieure à la température ambiante lorsque $R^2$ représente un radical alkyle.

En fin d'opération on obtient, dans le cas du polystyrène un matériau comportant des groupements de formule suivante :

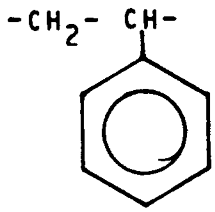

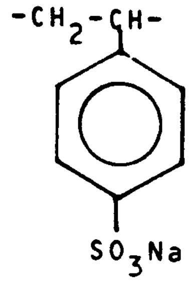

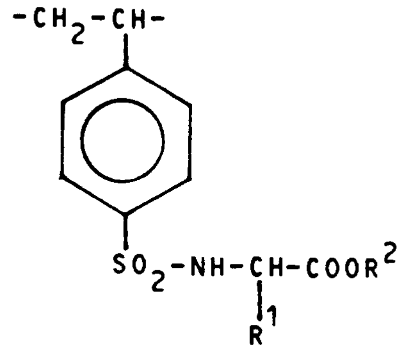

Le nombre de groupements substitués par $SO_3Na$ et par

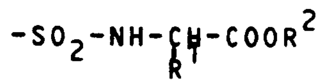

dépend en particulier des conditions de réaction.

Pour obtenir de bons résultats, il est préférable que le nombre de groupes styrène substitués par les groupements $-SO_2Y$ soit compris entre 10 et 30%.

Lorsque l'on utilise dans l'invention un support en copolymère de styrène obtenu par greffage de styrène sur un polymère résistant à la chlorosulfonation, on réalise de préférence le greffage par irradiation au moyen de rayonnements ionisants. Dans ce cas, on peut immerger une poudre du polymère résistant à la chlorosulfonation dans une solution de styrène, et soumettre l'ensemble à une irradiation en atmosphère exempte d'oxygène en utilisant par exemple comme source de radiations, une source de cobalt 60.

On contrôle le taux de greffage de la poudre en agissant sur la concentration en styrène de la solution, sur le type de solvant, sur le temps de diffusion, sur la dose totale d'irradiation et sur le débit de dose d'irradiation.

Après irradiation, on soumet généralement le produit greffé à un lavage réalisé par exemple au moyen de styrène puis d'alcool et on le sèche.

Ce mode de préparation de la poudre greffée est particulièrement avantageux car il permet d'obtenir un greffage du styrène à la surface de la poudre et de conférer à celle-ci après fixation des groupements appropriés une bonne affinité et une bonne sélectivité pour les anticorps AntiVIII:C.

Le procédé de l'invention peut être utilisé en particulier pour traiter des liquides constitués par du plasma sanguin, en particulier du plasma sanguin provenant de patients atteints d'hémophilie A. Dans ce cas, on peut utiliser le procédé de l'invention pour l'épuration ex-vivo du sang de patients de ce type.

D'autres caractéristiques et avantages de l'invention apparaîtront mieux à la lecture de la description qui suit donnée bien entendu à titre illustratif et non limitatif en référence au dessin annexé sur lequel :

- la figure 1 représente la courbe standard permettant de déterminer les concentrations en anticorps AntiVIII:C d'échantillons à partir des temps partiels de thromboplastine (PTT), et
- la figure 2 représente les isothermes d'adsorption des immunoglobulines IgG et des anticorps AntiVIII:C sur le support, de l'exemple 1.

EXEMPLE 1 : Préparation d'un support en polystyrène comportant des groupements $-SO_2Glu$ et $-SO_3Na$.

a) Préparation du polystyrène chlorosulfoné.

On laisse gonfler trois heures à la température ambiante 18 g de billes de polystyrène réticulé ayant une granulométrie de 40 à 70 µm dans 1440 ml de dichlorométhane. On ajoute alors un mélange de 270

ml de dichlorométhane et de 227 ml d'acide chlorosulfonique. On agite la suspension pendant 25 min. à la température ambiante, puis on filtre la résine brute, on la lave avec précaution avec des mélanges dichlorométhane-dioxanne et on la sèche finalement sous vide à 50 C.

On détermine alors le taux de groupes chlorosulfonyle ($-SO_2Cl$) de la façon suivante : 200 mg de polystyrène chlorosulfoné sont hydrolysés avec 50 ml d'une solution 1M de NaOH pendant 24 h au reflux. Après acidification, les ions $Cl^-$ libérés sont titrés par une solution 0,1 M de $AgNo_3$ en utilisant une électrode indicatrice d'argent.

b) Fixation d'acide glutamique sur les fonctions chlorosulfonyle

On prépare une solution d'acide glutamique dans un solvant constitué par un mélange d'eau et de dioxane dans un rapport 3/2 et on ajuste le pH de la solution à 9-10 par addition de soude 4M pour dissoudre l'acide glutamique. On ajoute alors 10 g du polystyrène chlorosulfoné obtenu précédemment et on maintient le pH à sa valeur initiale de 9-10 par addition de soude 2M. La réaction est terminée lorsque le pH reste stable. On filtre alors le polymère, on le lave abondamment à l'eau, puis avec de la soude $10^{-2}$M et de l'eau, et on le sèche sous vide.

On détermine alors la teneur en groupes $-SO_2Glu$ du polymère obtenu par analyse élémentaire de la teneur en azote. On détermine également la teneur en groupes $SO_3Na$ à partir de la teneur en groupes chlorosulfonyle déterminée précédemment et de la teneur en groupes $-SO_2Glu$. Les résultats obtenus sont donnés dans le tableau joint.

EXEMPLES 2 et 3. Préparation de supports en polystyrène comportant des groupements $-SO_2OHPro$ et $-SO_2Threo$.

On utilise du polystyrène chlorosulfoné tel que celui obtenu dans l'exemple 1 a), puis on fixe sur celui-ci l'acide aminé correspondant, soit, l'hydroxyproline (ex.2) ou la thréonine (ex.3), en utilisant le même mode opératoire que dans l'exemple 1 b). On détermine comme précédemment les teneurs en groupements $-SO_2Y$ et $-SO_3Na$ des supports ainsi obtenus. Les résultats sont donnés dans le tableau joint.

EXEMPLE 4. Cet exemple illustre l'utilisation des supports des exemples 1 à 3 pour adsorber les anticorps AntiVIII:C présents dans le plasma d'un patient hémophile.

a) Conditionnement et lavage des supports

Les supports des exemples 1 à 3 ont tout d'abord été conditionnés afin d'éliminer totalement toute impureté qui pourrait réagir avec les protéines du sang.

Dans ce but, on lave successivement les supports avec une solution de chlorure de sodium 1,5 M et des solutions de citrate de sodium 1,0 M. On équilibre ensuite ce support dans du tampon de Michaelis (pH : 7,35), puis on filtre, on lave plusieurs fois avec de l'eau et on sèche sous vide. Après broyage, les dimensions moyennes des particules des supports gonflés à l'eau, déterminées en utilisant un microscope quantitatif du type TAS sont dans l'intervalle de 5 à 10 $\mu$m.

b) Préparation des échantillons

On prépare tout d'abord des échantillons de plasma, d'une part, à partir du sang d'un patient hémophile ayant des anticorps AntiVIII:C et, d'autre part, à partir du sang recueilli sur 15 sujets normaux servant de référence. Le sang est collecté sur une solution de citrate de trisodium à 3,8%, à raison d'un volume de solution de citrate pour neuf volumes de sang, puis stocké à une température de -70 °C.

On isole ensuite les immunoglobulines du plasma du sujet hémophile dont le titre en anticorps AntiVIII:C dans le plasma est de 640 u Bethesda/ml. Dans ce but, le plasma de l'hémophile en question est défibrinné puis dialysé contre un tampon au phosphate (0,005M, pH = 6,5) pendant une nuit, à 4 °C.

On fait passer ensuite le sérum obtenu sur une colonne de cellulose DEAE 52 et on recueille les fractions contenant les immunoglobulines IgG, puis on les concentre et on les dialyse abondamment contre une solution de NaCl 0,15 M. La préparation finale d'immunoglobulines G provenant du patient hémophile ($IgG_H$) présente une activité en anticorps AntiVIII:C de 50 u Bethesda/mg de IgG.

la concentration en anticorps AntiVIII:C est déterminée selon la méthode décrite par La conférence de Bethesda C.K. KASPER et col. "A more uniform measurement of Factor VIII inhibitors" Thromb. Diath. Haemorrh, Vol. 34, p. 869 (1975). Selon cette méthode, on fait incuber 0,1 ml de la préparation de plasma

contenant de l'AntiVIII:C ou de la préparation d'IgG avec 0,1 ml de plasma normal pendant deux heures à 37° C. On détermine ensuite l'activité procoagulante du facteur VIII du mélange et on la compare à celle d'un tube de contrôle dans lequel du tampon Michaelis a été incubé avec du plasma normal. L'activité procoagulante du facteur VIII est mesurée à partir du temps partiel de thromboplastine (PTT) en utilisant du plasma humain déficitaire en facteur VIII comme substrat et du plasma normal préparé comme décrit ci-dessus comme standard (1u/ml). L'unité d'activité AntiVIII:C est définie comme celle qui inactive 50% de l'activité procoagulante de l'échantillon témoin pendant l'incubation de deux heures. Ainsi, la concentration en anticorps AntiVIII:C, exprimée en unités Bethesda est obtenue en déterminant l'inverse du taux de dilution de la préparation de plasma témoin ou de la préparation d'IgG qui inactive 0,5 u de VIII:C pendant les deux heures d'incubation.

c) Essais d'adsorption

On réalise ces essais en faisant incuber 50 µl de préparations de $IgG_H$ à des concentrations variées, soit avec une suspension de l'un des supports des exemples 1 à 7 en utilisant 2 à 10 mg/ml de support, soit avec du tampon Michaelis. Après 30 min d'incubation, on centrifuge les mélanges contenant $IgG_H$ et on détermine ensuite les concentrations en IgG et/ou en anticorps AntiVIII:C des surnageants de la façon suivante :

1°) la concentration en IgG est mesurée par immunodiffusion radiale en utilisant des plaques ICL (plaques commercialisées par ICL Scientific, Fountain Valley, Californie). Les trousses de dosage pour taux faible ou pour taux ultrafaible ont été utilisées selon les concentrations de IgG à déterminer. Pour effectuer les mesures, on traite sur la même plaque deux échantillons standard ayant des concentrations connues en immunoglobuline IgG qui sont respectivement de 0,15 et 3 mg/ml, avec 4 surnageants contenant $IgG_H$ et pour obtenir une reproductibilité maximale, on utilise une micropipette de précision afin de délivrer un volume de 5,0 µl de surnageant dans chaque puits. Par ailleurs, pour éviter une distorsion dans les résultats, les échantillons correspondant la concentration en IgG avant et après l'adsorption sont délivrés dans deux puits adjacents.

2°) La détermination quantitative en anticorps antiVIII:V est effectuée en utilisant une courbe standard qui met en corrélation le temps partiel de thromboplastine (PTT) avec les concentrations en anticorps AntiVIII:C. Cette courbe standard est obtenue à partir de préparations de $IgG_H$ ayant des concentrations en AntiVIII:C variant de 0 jusqu'à deux unités Bethesda/ml, qui sont obtenues par dilution de la préparation de $IgG_H$. Pour établir la courbe standard, on fait incuber les différentes dilutions de $IgG_H$ qui contiennent de l'AntiVIII:C, tout d'abord avec du plasma normal pendant deux heures à 37° C, puis après une dilution au 1/10°, on fait incuber 0,1 ml de ces mélanges avec 0,1 ml de plasma d'hémophile et 0,1 ml de céphaline activée pendant 10 min. On mesure ensuite les temps partiels de thromboplastine (PTT) après avoir ajouté 0,1 ml de $CaCl_2$.

On observe une élévation du PTT dés que la concentration en AntiVIII:C augmente. Les résultats obtenus sont donnés sur la figure 1 annexée qui illustre la courbe standard obtenue représentant le temps partiel de thromboplastine PTT (en secondes) en fonction de la concentration en AntiVIII:C (en u/ml).

Aprés avoir établi cette courbe standard, on mesure les temps partiels de thromboplastine PTT sur les surnageants et on détermine leur concentration en AntiVIII:C en utilisant la courbe standard et les facteurs de dilution.

Ces différentes mesures permettent de déterminer les concentrations initiales en IgG et en anticorps AntiVIII:C des échantillons avant adsorption, et les concentrations résiduelles en IgG et en anticorps AntiVIII:C des échantillons après leur mise en contact avec les supports adsorbants.

En effet, les concentrations en IgG et en AntiVIII:C des échantillons qui ont été mis en contact avec le tampon Michaelis correspondent respectivement aux concentrations initiales divisées par 2 en IgG et en AntiVIII:C des échantillons. Les concentrations en IgG et en AntiVIII:C des échantillons qui ont été mis en contact avec les supports adsorbants, correspondent aux concentrations résiduelles en IgG et en AntiVIII:C. Ainsi, on peut en déduire les taux de IgG et d'anticorps AntiVIII:C adsorbés sur les différents supports.

A partir des résultats obtenus sur différents échantillons, on peut construire les courbes d'adsorption isothermes de IgG et d'anticorps AntiVIII:C.

Sur la figure 2 on a représenté les isothermes d'adsorption de IgG et d'AntiVIII:C qui correspondent au support de l'exemple 1.

Sur cette figure, la courbe 1 correspond à l'adsorption de IgG et l'on a porté en abscisse les concentrations en IgG (en mg/ml) avant adsorption et en ordonnée les concentrations en IgG (mg/ml) adsorbées sur le support. La courbe 2 correspond à l'adsorption isotherme de l'AntiVIII:C et l'on a porté en abscisse les concentrations en AntiVIII:C (u/ml) avant adsorption et en ordonnée les concentrations en

AntiVIII:C (u/ml) adsorbées. La disposition des échelles qui tient compte de l'activité spécifique des $IgG_H$ permet la comparaison directe des deux types d'isothermes.

A partir de ces courbes, on peut déterminer la valeur $S_1$ du rapport de la pente de l'isotherme d'adsorption (2) relative à l'AntiVIII:C à la pente de l'isotherme d'adsorption (1) relative à IgG et la valeur $S_2$ du rapport entre les valeurs d'adsorption au plateau pour les deux isothermes précitées. Lorsque la sélectivité du support est nulle pour l'AntiVIII:C les deux types d'isothermes d'adsorption se superposent et $S_1$ et $S_2$ sont voisins de 1.

En revanche, lorsque la sélectivité est élevée, $S_1$ et $S_2$ ont des valeurs élevées.

Dans le tableau joint, on a reporté les valeurs de $S_1$ et/ou $S_2$ obtenues lors des essais d'adsorption sur les supports des exemples 1 à 3.

Sur ce tableau, on voit que de bonnes sélectivités sont obtenues avec les supports des exemples 1 à 3. Dans le cas du support de l'exemple 1, 60% des anticorps AntiVIII:C sont adsorbés alors que 16% seulement des IgG sont adsorbées, ce qui représente une bonne sélectivité.

EXEMPLE 5. Support en polystyrène sur lequel sont fixés uniquement des groupes $-SO_3Na$.

On prépare comme dans l'exemple 1 a) du polystyrène chlorosulfoné contenant 4 meq/g de groupements $-SO_2Cl$, et l'on hydrolyse ensuite les fonctions chlorosulfonyle en utilisant une solution de soude NaOH 2M à une température de 60 ° C. On immerge la poudre qui a été soumise au traitement de chlorosulfonation dans cette solution de soude pendant 24 heures, ce qui permet de transformer la totalité des fonctions $-SO_2Cl$ en groupements $-SO_3Na$.

On conditionne et on lave le support ainsi préparé comme dans l'exemple 4 a) et on réalise avec ce support des essais d'adsorption des anticorps AntiVIII:C présents dans le plasma d'un patient hémophile dans les mêmes conditions que celles de l'exemple 4.

A partir des isothermes d'adsorption obtenues dans les mêmes conditions que celles de l'example 4, on détermine les valeurs de $S_1$ et de $S_2$ et l'on trouve que $S_1 = 3,2$ et $S_2 = 2,7$. Ainsi, ce support présente, lui aussi, des propriétés intéressantes pour réaliser l'adsorption sélective des anticorps AntiVIII:C.

TABLEAU

| Acide aminé fixé | $SO_3Na$ meq/g | $SO_2Y$ * meq/g | $R^1$ | $R^2$ | $S_1$ | $S_2$ |
|---|---|---|---|---|---|---|
| Acide glutamique (ex. 1) | 3,2 | 0,8 | $-(CH_2)_2-COOH$ | H | 4,3 | 4,2 |
| Hydroxyproline (ex. 2) | 1,8 | 2,2 | 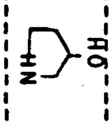 | H | 4,5 | 4,5 |
| thréonine (ex. 3) | 3 | 1 | $-CHOH-CH_3$ | H | 3,7 | 3,1 |

* $Y = NH-CH(R^1)-COOR^2$

## Revendications

**1.** Procédé de séparation des anticorps AntiVIII:C présents dans un liquide, caractérisé en ce qu'il consiste :

a)à mettre en contact ledit liquide avec un support solide constitué par un polymère ou un copolymère comportant dans sa chaîne des groupes substituables dont une partie au moins est substituée par des groupements de formule $-(SO_3)_xM$ et $-SO_2Y$, ou $-(SO_3)_xM$ uniquement, dans lesquelles M représente un métal ne réagissant pas avec le liquide et x représente la valence de M, et Y représente un radical obtenu par enlèvement d'un atome d'hydrogène sur la fonction amine d'un composé choisi parmi l'acide glutamique, l'hydroxyproline, la thréonine, et leurs dérivés, et
b)à séparer ensuite le liquide du support sur lequel ont été adsorbés les anticorps AntiVIII:C.

**2.** Procédé selon la revendication 1, caractérisé en ce que le radical Y est obtenu par enlèvement d'un atome d'hydrogène sur la fonction amine de l'acide glutamique.

**3.** Procédé selon la revendication 1, caractérisé en ce que le radical Y est obtenu par enlèvement d'un atome d'hydrogène sur la fonction amine de l'hydroxyproline.

**4.** Procédé selon la revendication 1, caractérisé en ce que le radical Y est obtenu par enlèvement d'un atome d'hydrogène sur la fonction amine de la thréonine.

**5.** Procédé selon l'une quelconque des revendications 1 à 4, caractérisé en ce que ledit support est constitué par du polystyrène ou par un copolymère de styrène sur lequel sont fixés des groupements de formule $-SO_2Y$ dans laquelle Y a la signification donnée dans l'une quelconque des revendication 1 à 4 et des groupements de formule $-(SO_3)_xM$ dans laquelle M représente un métal ne réagissant pas avec le liquide et x représente la valence du métal M.

**6.** Procédé selon la revendication 5, caractérisé en ce que le copolymère de styrène est constitué par un polymère résistant à la chlorosulfonation greffé par du styrène.

**7.** Procédé selon la revendication 6, caractérisé en ce que le polymère résistant à la chlorosulfonation est choisi parmi les polyoléfines, les polymères fluorés et le chlorure de polyvinyle.

**8.** Procédé selon l'une quelconque des revendications 1 à 7, caractérisé en ce que ledit liquide est du plasma sanguin.

**Claims**

**1.** Process for the separation of antiVIII:C antibodies present in a liquid, characterized in that it comprises:
a) contacting said liquid with a solid support constituted by a polymer or copolymer having substitutable groups in its chain and whereof at least part is substituted by groups of formula $-(SO_3)_xM$, and $-SO_2Y$ or $-(SO_3)_xM$ only, in which M represents a metal which does not react with the liquid and x represents the valency of M and Y represents a radical obtained by removing a hydrogen atom from the amino function of a compound chosen from among glutamic acid, hydroxyproline, threonine and their derivatives and
b) then separating the liquid from the support on which have been adsorbed the antiVIII:C antibodies.

**2.** Process according to claim 1, characterized in that the radical Y is obtained by removing a hydrogen atom from the amino function of the glutamic acid.

**3.** Process according to claim 1, characterized in that the radical Y is obtained by removing a hydrogen atom from the amino function of hydroxyproline.

**4.** Process according to claim 1, characterized in that the radical Y is obtained by removing a hydrogen atom from the amino function of threonine.

**5.** Process according to any one of the claims 1 to 4, characterized in that said support is constituted by polystyrene or a styrene copolymer to which are fixed groups of formula $-SO_2Y$, in which Y has the meaning given in any one of the claims 1 to 4 and groups of formula $-(SO_3)_xM$, in which M represents a metal which does not react with the liquid and x represents the valency of the metal M.

**6.** Process according to claim 5, characterized in that the styrene copolymer is constituted by a styrene-grafted, chlorosulphonation-resistant polymer.

**7.** Process according to claim 6, characterized in that the chlorosulphonation-resistant polymer is chosen from among polyolefins, fluorinated polymers and polyvinylchloride.

**8.** Process according to any one of the claims 1 to 7, characterized in that the liquid is blood plasma.

**Patentansprüche**

**1.** Verfahren zur Abtrennung der in einer Flüssigkeit enthaltenen Antikörper AntiVIII:C, dadurch gekennzeichnet, daß man:

(a) die genannte Flüssigkeit mit einem festen Träger in Kontakt bringt, der besteht aus einem Polymeren oder einem Copolymeren, das in seiner Kette substituierbare Gruppen aufweist, von denen mindestens ein Teil substituiert ist durch Gruppierungen der Formel $-(SO_3)_xM$ und $-SO_2Y$ oder nur $-(SO_3)_xM$, worin darstellen M ein Metall, das mit der Flüssigkeit nicht reagiert, x die Valenz von M und Y einen Rest, der erhalten wird, wenn man aus der Aminfunktion einer Verbindung, ausgewählt aus Glutaminsäure, Hydroxyprolin, Threonin und ihren Derivaten, ein Wasserstoffatom entfernt, und

(b) anschließend die Flüssigkeit von dem Träger trennt, an dem die Antikörper AntiVIII:C adsorbiert worden sind.

**2.** Verfahren nach Anspruch 1, dadurch gekennzeichnet, daß der Rest Y erhalten wird durch Entfernung eines Wasserstoffatoms aus der Aminfunktion der Glutaminsäure.

**3.** Verfahren nach Anspruch 1, dadurch gekennzeichnet, daß der Rest Y erhalten wird durch Entfernung eines Wasserstoffatoms aus der Aminfunktion des Hydroxyprolins.

**4.** Verfahren nach Anspruch 1, dadurch gekennzeichnet, daß der Rest Y erhalten wird durch Entfernung eines Wasserstoffatoms aus der Aminfunktion des Threonins.

**5.** Verfahren nach einem der Ansprüche 1 bis 4, dadurch gekennzeichnet, daß der genannte Träger besteht aus Polystyrol oder aus einem Copolymeren von Styrol, an dem Gruppierungen der Formel $-SO_2Y$, worin Y die in einem der Ansprüche 1 bis 4 angegebene Bedeutung hat, und Gruppierungen der Formel $-(SO_3)_xM$ fixiert sind, worin M ein Metall, das mit der Flüssigkeit nicht reagiert, und x die Valenz des Metalls M darstellen.

**6.** Verfahren nach Anspruch 5, dadurch gekennzeichnet, daß das Styrol-Copolymere besteht aus einem gegen Chlorsulfonierung beständigen Polymeren mit aufgepfropftem Styrol.

**7.** Verfahren nach Anspruch 6, dadurch gekennzeichnet, daß das gegen Chlorsulfonierung beständige Polymere ausgewählt wird aus den Polyolefinen, den fluorierten Polymeren und Polyvinylchlorid.

**8.** Verfahren nach einem der Ansprüche 1 bis 7, dadurch gekennzeichnet, daß die genannte Flüssigkeit Blutplasma ist.

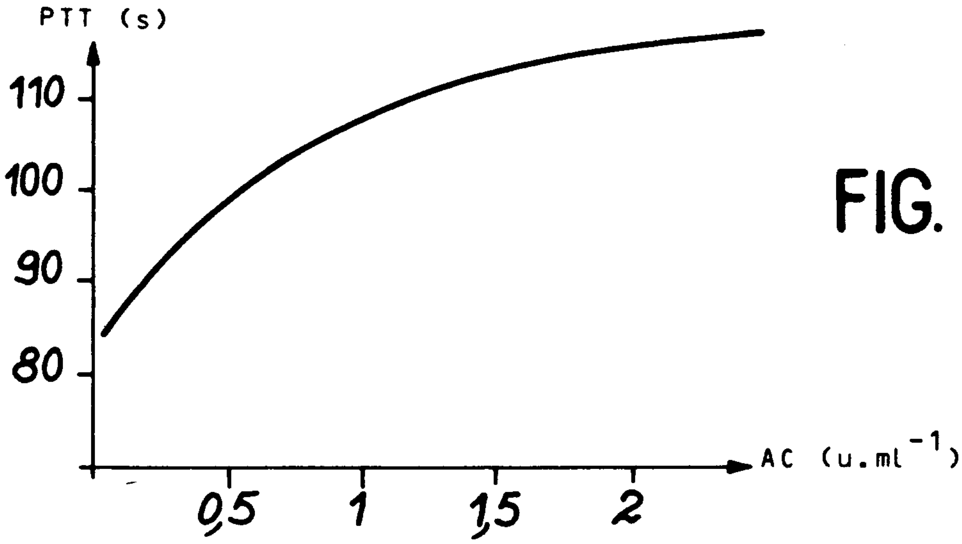
PTT (s)
110
100
90
80
AC (u.ml$^{-1}$)
0,5
1
1,5
2

FIG. 1

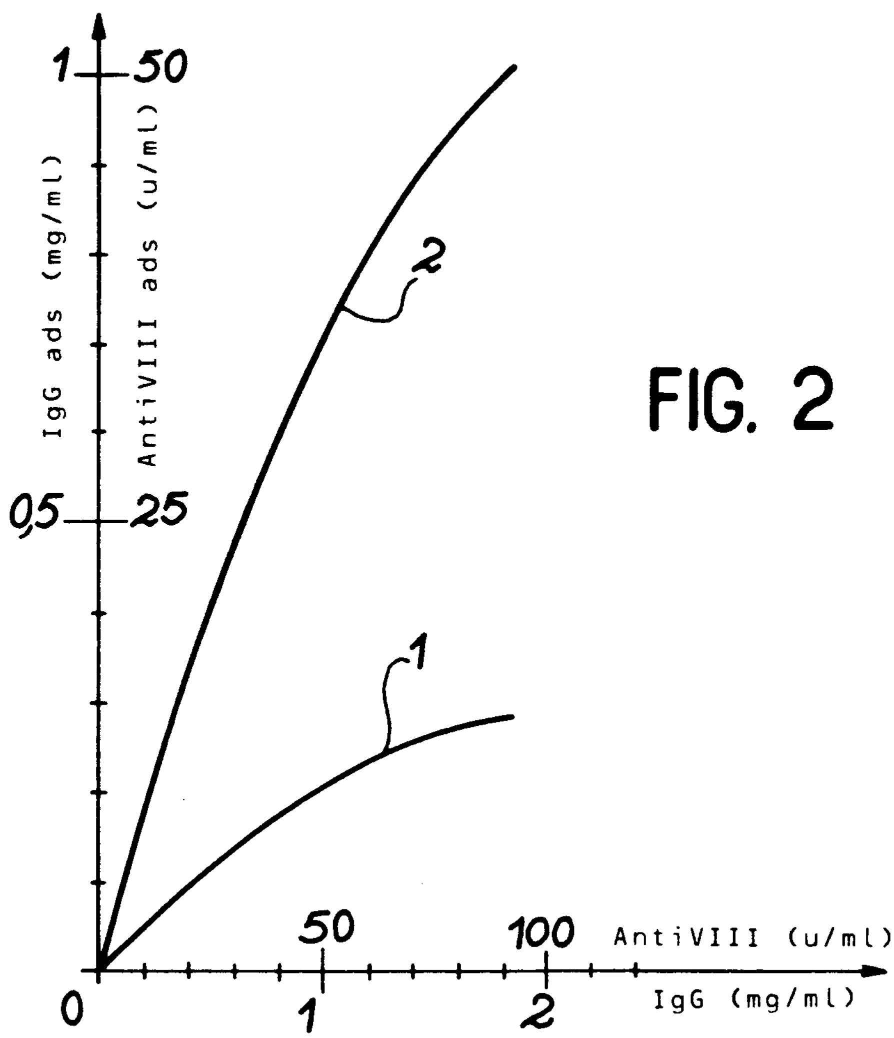
1
50
0,5
25
IgG ads (mg/ml)
AntiVIII ads (u/ml)
2
1
50
100
AntiVIII (u/ml)
0
1
2
IgG (mg/ml)

FIG. 2